**Europäisches Patentamt**

**European Patent Office**

**Office européen des brevets**

(19)

(11) Veröffentlichungsnummer: **0 027 917**
**B1**

(12) # EUROPÄISCHE PATENTSCHRIFT

(45) Veröffentlichungstag der Patentschrift:
10.10.84

(51) Int. Cl.³: **G 01 N 31/22, G 01 N 33/18**

(21) Anmeldenummer: **80105954.4**

(22) Anmeldetag: **02.10.80**

(54) Reagenz und Verfahren zur Bestimmung von Hydrazin.

(30) Priorität: 24.10.79 DE 2942960

(43) Veröffentlichungstag der Anmeldung:
06.05.81 Patentblatt 81/18

(45) Bekanntmachung des Hinweises auf die Patenterteilung:
10.10.84 Patentblatt 84/41

(84) Benannte Vertragsstaaten:
DE FR GB NL

(56) Entgegenhaltungen:
CHEMICAL ABSTRACTS, Band 83, 1975, Columbus, Ohio, USA M. MATSUTANI "Measuring content of urea nitrogen in serum", Seite 270, Zusammenfassung Nr. 175 162v
CHEMICAL ABSTRACTS, Band 83, 1975, Columbus, Ohio, USA E.J. DIXON et al. "Determination of unsulfonated primary aromatic amines in water-soluble food dyes and other food additives", Seite 380, Zusammenfassung Nr. 130 105n
Ullmanns Encyklopädie der technischen Chemie, 4. Auflage, Band 8, Seite 203.
The Merck Index, Ninth Edition, 1976, Seite 494.

Die Akte enthält technische Angaben, die nach dem Eingang der Anmeldung eingereicht wurden und die nicht in dieser Patentschrift enthalten sind.

(73) Patentinhaber: **Merck Patent Gesellschaft mit beschränkter Haftung, Frankfurter Strasse 250, D-6100 Darmstadt (DE)**

(72) Erfinder: **Bodart, Detlef, Dr., Karlstrasse 56, D-6100 Darmstadt (DE)**
Erfinder: **Bitsch, Roland, Karl-Marx-Strasse 40, D-6102 Pfungstadt (DE)**

**Beschreibung**

Die Erfindung betrifft ein stabiles Reagenz und ein Verfahren zur Bestimmung von Hydrazin in wäßrigen Lösungen.

Hydrazin ist ein bekanntes Korrosionsschutzmittel für Stahl im Kontakt mit Wasser, z. B. im Dampfkesselbetrieb, in Warmwasserumlaufsystemen, der Stillstandkonservierung von Behältern usw. Dabei werden dem Wasser je nach den Temperatur- und Druckverhältnissen 0,1—300 ppm Hydrazin zugegeben, wobei Systeme mit höheren Drücken und Temperaturen die geringsten Hydrazinmengen erfordern. Durch den Hydrazinverbrauch während des Dampfkesselbetriebs sinken die zu messenden Hydrazingehalte oft unter 0,1 ppm ab. Diese geringen Konzentrationen können mit einfachen Schnelltests bisher nicht gemessen werden. Der Resthydrazingehalt im Bereich unter 0,1 ppm stellt jedoch im Kesselbetrieb einen wichtigen Parameter dar, da dieser den Zeitpunkt und das Ausmaß der Hydrazinnachdosierung bestimmt.

Zur Bestimmung von Hydrazin im Wasser gibt es eine Standardmethode, die seit Einführung im Jahre 1947 die Basis aller Betriebslabor- und Schnelltest-Verfahren bildet. Es handelt sich dabei um die Reaktion von Hydrazin mit 4-Dimethylaminobenzaldehyd zu 4-Dimethylaminobenzaldehyd-azin (Bull. Soc. Chim. France, 122—123 (1947)).

Andererseits ist bekannt, daß primäre und sekundäre aromatische Amine in Methanol in Gegenwart von Trichloressigsäure mit 4-Dimethylaminozimtaldehyd eine empfindlichere und tiefere Färbung geben als mit 4-Dimethylaminobenzaldehyd (Chemical Abstracts 55, 1961bb (1960)).

Auch ist ein Verfahren zur Bestimmung des Harnstoff-Stickstoffs im Serum bekannt, bei dem dieser durch eine Farbreaktion des Harnstoff-Amins mit 4-Dimethylaminobenzaldehyd in salzsauer-ethanolischer Lösung durch Messung der Absorption bei 520 nm bestimmt wird (Chemical Abstracts 83, 175162v (1975)).

Die etablierte und zuverlässige Methode der Hydrazinbestimmung mit 4-Dimethylaminobenzaldehyd hat jedoch den entscheidenden Nachteil, daß die Lösung des 4-Dimethylaminobenzaldehyds gelb ist und das entsprechende sich bildende Azin ebenfalls eine, wenn auch etwas kräftigere, gelbe Färbung besitzt. Da das Auge gelbe Farbintensitätsunterschiede schlechter als solche anderer Farben differenzieren kann, waren bei optischen Tests bisher nur relativ grobe Abstufungen der Hydrazinkonzentrationen möglich, z. B. von 0—0,1—0,25—0,5—1,0 ppm Hydrazin. Besondere Schwierigkeiten traten bei niedrigen Hydrazingehalten auf, da diese durch Abstufungen schwacher Gelbtöne bei Vorlage eines bereits gelb gefärbten Blindwertes schwer zuzuordnen waren. Ein weiteres Problem ist die optische Messung bei Kunstlicht, wo die Differenzierbarkeit von Gelbtönen generell nachläßt.

Andererseits weist im Gegensatz zu 4-Dimethylaminobenzaldehyd der 4-Dimethylaminozimtaldehyd eine schlechte Haltbarkeit in Lösung auf. Eine gute Haltbarkeit ist jedoch für ein flüssiges Fertigreagenz unbedingt erforderlich, um nicht vor jeder Bestimmung eine frisch angesetzte Reagenzlösung herstellen zu müssen.

Der Erfindung lag deshalb die Aufgabe zugrunde, ein stabiles, empfindliches Reagenz und ein Verfahren zur Bestimmung von Hydrazin in wäßrigen Lösungen zur Verfügung zu stellen, mit dem geringste Hydrazingehalte noch sicher erfaßt werden können.

Überraschenderweise wurde gefunden, daß eine 4-Dimethylaminozimtaldehyd-Lösung in Äthylenglycolmonoalkyläther eine sehr gute Haltbarkeit aufweist, und daß mit Hilfe dieser Lösung genaue und reproduzierbare Analysenergebnisse auch im Bereich unter 0,1 ppm Hydrazin erhalten werden.

Gegenstand der Erfindung ist ein Mittel zur Bestimmung von Hydrazin in wäßrigen Lösungen in Form einer 2- bis 4%igen Lösung von 4-Dimethylaminozimtaldehyd in Äthylenglycolmonoalkyläther, insbesondere Äthylenglycolmonoäthyläther.

Ein weiterer Gegenstand der Erfindung ist ein Verfahren zur Bestimmung von Hydrazin in wäßrigen Lösungen, das dadurch gekennzeichnet ist, daß man der hydrazinhaltigen Probelösung eine 2—4%ige Lösung von 4-Dimethylaminozimtaldehyd in Äthylenglycolmonoalkyläther zusetzt und den Gehalt an gebildetem 4-Dimethylaminozimtaldehyd-azin colorimetrisch bestimmt.

Die Steigerung des Reaktionskontrasts mit Hilfe des erfindungsgemäßen Mittels führt zu Farbserien von gelb über orange nach weinrot, die eine zehnfache Abstufung ermöglichen, und sowohl bei Tageslicht als auch bei Kunstlicht optisch gleichermaßen gut gemessen werden können. Auch bei der photometrischen Messung ist der hohe Reaktionskontrast ($\Delta\lambda$) von 135 nm gegenüber der herkömmlichen Methode mit 4-Dimethylaminobenzaldehyd von nur 45 nm von Vorteil. Da das Extinktionsmaximum von 540 nm im Rotbereich liegt und durch eine gelbliche Wassereigenfarbe kaum beeinflußt wird, erübrigen sich die in diesen Fällen notwendigen Farbkompensationsproben, in denen das Hydrazin in einem zusätzlichen Ansatz beseitigt werden muß.

Die Bestimmung von Hydrazin erfolgt in der Weise, daß man die Probelösung ansäuert und mit der Lösung von 4-Dimethylaminozimtaldehyd in Äthylenglycolmonoalkyläther versetzt. Zum Ansäuern können alle Säuren verwendet werden, die die Bestimmung nicht stören, vorzugsweise Mineralsäuren wie Schwefelsäure, Salzsäure, Perchlorsäure, Phosphorsäure usw. Als besonders geeignet haben sich Gemische von Schwefelsäure mit Perchlorsäure erwiesen. Nach guter Durchmischung von Probe- und Reagenzlösung erfolgt nach 5 bis 20 Minuten die visuelle oder spektrophotometrische Auswertung. Erfolgt die Bestimmung in einem Rundbodenglas, so wird die visuelle Auswertung anhand einer Farbskala vorgenommen; dabei können noch 0,01 ppm Hydrazin sicher nachgewiesen werden. Wird die Bestimmung in einer Küvette ausgeführt, so erfolgt die spektrophotometrische Messung bei 540 nm.

### Beispiel 1

In einem Rundbodenglas von 24 mm Durchmesser werden zu 5 ml einer Lösung, die zwischen 0,2 und 4 ppm Hydrazin enthalten kann, 0,2 ml eines Schwefelsäure/Perchlorsäure-Gemischs (7 Teile 25%ige Schwefelsäure und 3 Teile 70%ige Perchlorsäure) sowie 0,15 ml einer 3%igen Lösung von 4-Dimethylaminozimtaldehyd in Äthylenglycolmonoäthyläther hinzugegeben. Nach guter Durchmischung und einer Standzeit von etwa 10 Minuten wird das Glas über einer weißen Unterlage fixiert; von oben gesehen ist eine deutliche Farbabstufung von gelb nach rot zu erkennen, die anhand einer Farbskala eindeutig von Hydrazingehalten zuordnet werden können.

### Beispiel 2

Zu 20 ml einer Lösung, die zwischen 0,02 und 0,25 ppm Hydrazin enthalten kann, kommen 0,4 ml eines Schwefelsäure/Perchlorsäure-Gemischs (7 Teile 25%ige Schwefelsäure und 3 Teile 70%ige Perchlorsäure) sowie 0,3 ml einer 3%igen Lösung von 4-Dimethylaminozimtaldehyd in Äthylenglycolmonoäthyläther. In einem auf einer weißen Unterlage stehenden Flachbodenglas von 20 mm Durchmesser ergibt dies von oben gesehen eine deutliche Farbabstufung von gelb nach orangerot. Anhand einer Farbskala lassen sich die Hydrazingehalte exakt bestimmen.

### Beispiel 3

Zu 5 ml einer Lösung, die zwischen 0,05 und 4,5 ppm Hydrazin enthalten kann, werden 0,2 ml eines Schwefelsäure/Perchlorsäure-Gemischs sowie 0,15 ml einer 3%igen Lösung von 4-Dimethylaminozimtaldehyd in Äthylenglycolmonoäthyläther hinzugegeben. Nach 10 Min. Standzeit wird die Färbung in einer Küvette von 10 mm Schichtdicke bei 540 nm im Spektraphotometer gegen eine gleichzeitig zubereitete Blindprobe gemessen. Die Konzentration an Hydrazin wird anhand des gemessenen Extinktionswertes an einer zuvor mit Hydrazinlösungen genau bekannten Gehaltes erstellten Eichkurve abgelesen.

## Patentansprüche

1. Mittel zur Bestimmung von Hydrazin in wäßrigen Lösungen, dadurch gekennzeichnet, daß es in Form einer 2- bis 4%igen Lösung von 4-Dimethylaminozimtaldehyd in Äthylenglycolmonoalkyläther vorliegt.

2. Mittel nach Anspruch 1, dadurch gekennzeichnet, daß es aus einer Lösung von 4-Dimethylaminozimtaldehyd in Äthylenglycolmonoäthyläther besteht.

3. Verfahren zur Bestimmung von Hydrazin in wäßrigen Lösungen, dadurch gekennzeichnet, daß man der Probelösung das Mittel nach den Ansprüchen 1 und 2 zusetzt und den Gehalt an gebildetem 4-Dimethylaminozimtaldehyd-azin colorimetrisch bestimmt.

## Claims

1. Agent for the determination of hydrazine in aqueous solutions, characterized in that it is in the form of a 2 to 4% solution of 4-dimethylaminocinnamaldehyde in an ethylene glycol monoalkyl ether.

2. Agent according to claim 1, characterized in that it consists of a solution of 4-dimethylaminocinnamaldehyde in ethylene glycol monoethyl ether.

3. Method for the determination of hydrazine in aqueous solutions, characterized in that it comprises adding to the sample solution the agent according to claims 1 and 2 and determining the content of the 4-dimethylaminocinnamaldehyde azine formed by colorimetry.

## Revendications

1. Réactif pour le dosage de l'hydrazine dans des solutions aqueuses, caractérisé en ce qu'il consiste en une solution à une concentration de 2 à 4% d'aldéhyde 4-diméthylaminocinnamique dans un éther monoalkylique de l'éthylène-glycol.

2. Réactif selon la revendication 1, caractérisé en ce qu'il consiste en une solution d'aldéhyde 4-diméthylaminocinnamique dans l'éther monoéthylique de l'éthylène-glycol.

3. Procédé pour doser l'hydrazine dans des solutions aqueuses, caractérisé en ce que l'on ajoute à la solution échantillon le réactif selon les revendications 1 et 2 et on détermine par colorimétrie la teneur en azine de l'aldéhyde 4-diméthylaminocinnamique formée.